# EUROPEAN PATENT SPECIFICATION

(11) **EP 2 940 132 B1**
(45) Date of publication and mention of the grant of the patent: **22.08.2018**
(21) Application number: 13867673.9
(22) Date of filing: 19.12.2013
(51) Int. Cl.: C12N 15/113, A61K 31/7088

(54) **SIRNA HAVING OBESITY PREVENTIVE OR THERAPEUTIC ACTIVITY**
SIRNA MIT WIRKUNG ZUR VERHINDERUNG ODER BEHANDLUNG VON ADIPOSITAS
SIRNA PRÉSENTANT UNE ACTIVITÉ PRÉVENTIVE OU THÉRAPEUTIQUE CONTRE L'OBÉSITÉ

(30) Priority: 26.12.2012 JP 2012283765; 22.07.2013 JP 2013152095
(43) Date of publication of application: 04.11.2015
(73) Proprietor: Nakajima, Toshihiro, Aoba-ku Yokohama-shi Kanagawa 225-0023 (JP)
(72) Inventor: NAKAJIMA Toshiro, Yokohama-shi Kanagawa 225-0023 (JP); FUJITA Hidetoshi, Chofu-shi Tokyo 182-0006 (JP); ARATANI Satoko, Tokyo 168-0062 (JP); YAGISHITA Naoko, Yokohama-shi Kanagawa 234-0054 (JP)
(74) Representative: Peters, Sebastian Martinus
(86) International application number: PCT/JP2013/084051
(87) International publication number: WO 2014/103863

(56) References cited:
- EP-A1- 1 709 973
- EP-A1- 2 725 106
- EP-A1- 2 954 905
- WO-A1-02/052007
- WO-A1-2005/074988
- WO-A1-2005/074988
- WO-A1-2006/137514
- WO-A1-2010/024852
- WO-A1-2010/024852
- WO-A1-2012/176860
- JP-A- 2009 155 204
- KR-A- 20090 089 094
- TOSHIHIKO IZUMI ET AL: "Activation of synoviolin promoter in rheumatoid synovial cells by a novel transcription complex of interleukin enhancer binding factor 3 and GA binding protein [alpha]", ARTHRITIS & RHEUMATISM, vol. 60, no. 1, 1 January 2009 (2009-01-01), pages 63-72, XP055233269, US ISSN: 0004-3591, DOI: 10.1002/art.24178
- TOSHIHIRO NAKAJIMA ET AL.: 'E3 Ubiquitin Ligase Synoviolin ni yoru Zoki Sen'ika Hassho no Bunshi Mechanism' DAIICHI SANKYO SEIMEI KAGAKU KENKYU SHINKO ZAIDAN KENKYU HOKOKUSHO vol. 28, 20 December 2012, pages 191 - 196, XP008180117
- IZUMI T ET AL.: 'Activation of synoviolin promoter in rheumatoid synovial cells by a novel transcription complex of interleukin enhancer binding factor 3 and GA binding protein alpha' ARTHRITIS RHEUM. vol. 60, no. 1, 2009, pages 63 - 72, XP055233269
- YAMASAKI S ET AL.: 'Cytoplasmic destruction of p53 by the endoplasmic reticulum-resident ubiquitin ligase 'Synoviolin' EMBO J. vol. 26, no. 1, 2007, pages 113 - 122, XP055233265
- TSUCHIMOCHI K ET AL.: 'Identification of a crucial site for synoviolin expression' MOL. CELL . BIOL. vol. 25, no. 16, 2005, pages 7344 - 7356, XP002433313

## Description

### TECHNICAL FIELD

The present invention relates to a therapeutic agent for use in treatment or prevention of obesity.

### BACKGROUND ART

Obesity causes excessive accumulation of adipose tissue and increases risk of various health problems, such as diabetes, cardiovascular diseases, and depression (see Non-Patent Literature 1). These problems lead to tremendous economic and social loss in modern society. Molecular mechanisms of adipocyte metabolism are extensively studied (see Non-Patent Literatures 2 and 3).

Synoviolin is a protein discovered as a membrane protein overexpressed in rheumatoid patient-derived synovial cells (see Patent Document 1). Studies using genetically modified animals have revealed that synoviolin is an essential molecule for the onset of rheumatoid arthritis.

Synoviolin has been suggested to have a RING finger motif based on analyses using a protein structure prediction system. This motif is found in large numbers in an enzyme known as E3 ubiquitin ligase, which plays an important role in protein ubiquitination. Synoviolin has been demonstrated to have self-ubiquitination activity, which is a characteristic of E3 ubiquitin ligase (see Patent Document 1).

### PRECEDING TECHNICAL DOCUMENT

### Patent Document

Patent Document 1: WO 02/052007.
Patent Document 2: WO 2005/074988 A1 describes a nerve cell differentiation induction drug containing a synoviolin expression inhibitor.
Patent Document 3: EP1709973 A1 describes a pharmaceutical composition comprising a substance that inhibits synoviolin expression and/or function that promotes activity of tumor suppressor gene p53 or protein p53.
Patent Document 4: KR20090089094 A describes the provision of a gene or protein of synoviolin to obtain a kit for diagnosing temporomandibular joint disorder (TMJD) and to screen a material for treating or preventing TMJD.
Patent document 5: WO2010/024852 A1 describes gene expression profiles associated with improved or maintained lean body mass or reduced body fat.

### Non Patent Literature:

Non-Patent Literature 1: Wickelgren, I. (1998). Obesity: how big a problem? Science 280, 1364-1367.
Non-Patent Literature 2: Lefterova, M.I., and Lazar, M.A. (2009). New developments in adipogenesis. Trends Endocrinol Metab 20, 107-114.
Non-Patent Literature 3: Rosen, E.D., and MacDougald, O.A. (2006). Adipocyte differentiation from the inside out. Nat Rev Mol Cell Biol 7, 885-896.
Non-Patent Literature 4: Toshihiko Izumi et al. (ARTHRITIS & RHEUMATISM, Vol. 60, No. 1, January 2009, pp 63-72, describe activation of synoviolin promoter in rheumatoid synovial cells by a novel transcription complex of interleukin enhancer binding factor 3 and GA binding protein α.

### SUMMARY OF THE INVENTION

### Technical Problem

A development of a screening method for a substance or a candidate compound thereof having an obesity preventive or therapeutic action is sought.

### Solution to Problem

Inventors of the present invention obtained an example-based knowledge that destruction of synoviolin gene causes reduction of body weight of a target and inhibition of differentiation induction of adipocytes, by using synoviolin gene conditional knockout mice. The inventors of the present invention found that compounds having regulating actions of a differentiation induction of adipocytes, an amount of adipose tissues, and body weight can be screened by screening compounds that inhibit synoviolin activity. The present invention has been completed by these findings.

A screening method for a substance having an anti-obesity action is described here. The method includes a step for contacting a test substance and a synoviolin-gene-expressing cell, and a step for verifying an effect of the test substance on synoviolin gene expression or on synoviolin protein activity. Examples of the effect on the synoviolin gene expression include an effect on a synoviolin mRNA level or an effect on a self-ubiquitination of synoviolin protein. The screening method for a substance having an anti-obesity action is a method for searching a candidate substance having the anti-obesity action.

In the screening method, the anti-obesity action is an action of reducing an amount of adipose tissues, or an action of inhibiting a differentiation induction of adipocytes. Namely, according to this method, a compound having the action of reducing the amount of adipose tissues or the action of inhibiting the differentiation induction of adipocytes can be screened.

The present invention relates to an anti-obesity drug, namely the therapeutic agent as defined in claim 1. The anti-obesity drug contains a decoy nucleic acid of synoviolin as an active ingredient thereof. The anti-obesity drug has, for example, an action of reducing an amount of adipose tissues, or an action of inhibiting a differentiation induction of adipocytes.

An example of synoviolin siRNA is a RNA having one base sequence selected from the following SEQ ID Nos. 2 to 6, complemental base sequences thereof, or a base sequence obtained by replacing, inserting, deleting, or adding one base or two bases with respect to one of these base sequences. The RNA having base sequences represented by the SEQ ID Nos. 2 to 4 are known as synoviolin siRNA as disclosed in Izumi T, et al., Arthritis Rheum. 2009; 60(1); 63-72., EMBO, Yamasaki S, et al., EMBO J. 2007; 26(1): 113-22. by using experiments. The RNA having base sequences represented by the SEQ ID Nos. 5 and 6 are known as synoviolin siRNA as disclosed in WO 2005/074988 by using experiments.

SEQ ID No. 2: 5' -GCUGUGACAGAUGCCAUCA-3'
SEQ ID No. 3: 5' -GGUGUUCUUUGGGCAACUG-3'
SEQ ID No. 4: 5' -GGUUCUGCUGUACAUGGCC-3'
SEQ ID No. 5: 5' -CGUUCCUGGUACGCCGUCA-3'
SEQ ID No. 6: 5' -GUUUTGGUGACUGGUGCUA-3'

The "RNA having a base sequence obtained by replacing, inserting, deleting, or adding one base or two bases with respect to one of these base sequences" is an RNA having a base sequence obtained by replacing, inserting, deleting, or adding one base or two bases with respect to the one base sequence selected from the SEQ ID Nos. 2 to 6 or the complemental base sequences thereof. One of replacement, insertion, deletion, and addition may be occurred, and two or more may be occurred.

The therapeutic agent of the present invention contains, as an active ingredient, a synoviolin decoy nucleic acid having a base sequence represented by SEQ ID No. 7 or a base sequence obtained by replacing, inserting, deleting, or adding one base or two bases with respect to the base sequence represented by SEQ ID No. 7. The nucleic acid having the base sequence represented by the SEQ ID No. 7 is known as synoviolin decoy nucleic acid as disclosed in Tsuchimochi K, et al., Mol Cell Biol.2005; 25(16): 7344-56 by examples.
SEQ ID No. 7: 5' -AUGGUGACUGGUGCUAAGA-3'

A therapeutic agent of which contains, as an active ingredient, a synoviolin antisense nucleic acid is also described herein.

A screening method is described herein for compounds having an obesity preventive or therapeutic action.

### BRIEF DESCRIPTION OF THE DRAWINGS

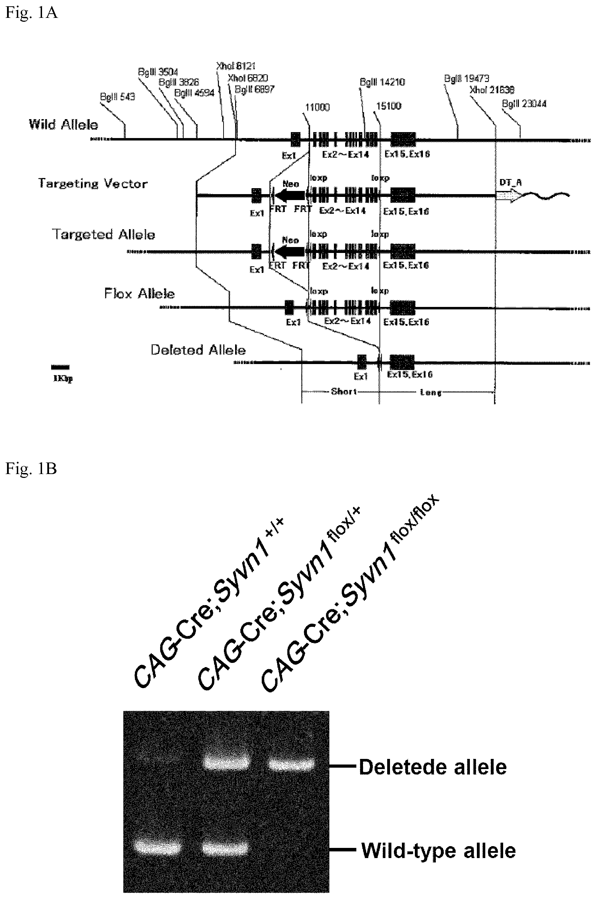

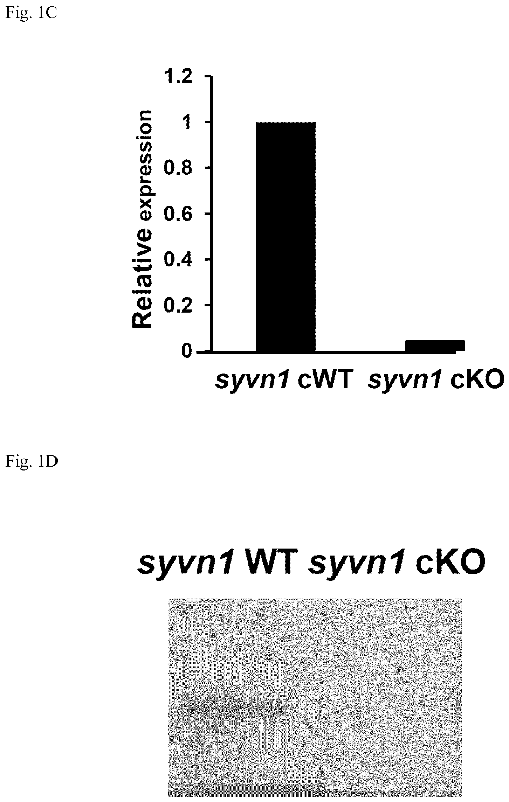

Fig. 1A shows a schematic depiction of a gene-targeting vector used for generation of synoviolin conditional knockout mouse. Exons are represented by black box, black arrow represents Neomycin (Neo), open arrow represents diphtheria toxin gene subunit A. Open triangles represent loxP sites, and Black circles represent FRT sites. Homologous gene recombination between endogenous synoviolin locus and the targeting vector resulted in a floxed chromosome carrying loxP flanked exons 2 to 12 and a FRT flanked Neo cassette. The floxed allele after excision of Neo is shown in Flox Allele. The Deleted allele was shown after tamoxifen (Tam)-induced Cre recombinase-mediated excision.
Fig. 1B shows a photograph of PCR analysis of genomic DNA from *(CAG)*-Cre-ER; *syvnl*^{+/+} mice, *(CAG)*-Cre-ER; *syvnl*^{flox/+} mice, and *(CAG)*-Cre-ER; syvnl^{flox/flox} mice after Tam treatment. The wild type allele is detected as a 70 bp fragment (lower band) and the targeted allele as 100 bp fragment (upper band).
Fig. 1C shows a graph of Real-time PCR analysis of adipocyte from *syvnl*^{flox//flox} mice *(syvnl* WT) and *(CAG)-*Cre-ER; *syvnl*^{flox/flox} mice *(syvnl* cKO) after Tam treatment.
Fig. 1D shows a Western blot of tail proteins from *syvnl* WT mice and *syvnl* cKO mice with anti-synoviolin antibody.
Fig. 1E shows a graph indicating a survival rate of postneonatal *synoviolin* conditional knockout mice.
Fig. 2A shows a graph indicating body weight changes in postneonatal *synoviolin* conditional knockout mice.
Fig. 2B shows graphs of food intakes of *synoviolin* knockout mice (male and female).
Fig. 2C shows photographs indicating a state of subcutaneous fat in postneonatal *synoviolin* conditional knockout mice. A white arrow represents the subcutaneous fat.
Fig. 2D shows a photograph of epididymal adipose tissue of *syvnl* WT mice (left) and *syvnl* cKO mice (right).
Fig. 2E shows photomicrographs indicating lipid droplets of visceral fat in postneonatal *synoviolin* conditional knockout mice. A black arrow represents the lipid droplets. Magnification is 400 folds.
Fig. 2F shows photomicrographs indicating lipid droplets of epididymal (upper diagrams) and subcutaneous fat (lower diagrams) in postneonatal *synoviolin* conditional knockout mice. A black arrow represents the lipid droplets. Magnification is 400 folds.
Fig. 3A shows a graph indicating body weight changes in syvnl cKO:ob/ob mice by postneonatal synoviolin knockout.
Fig. 3B shows photographs indicating a state of subcutaneous fat in syvnl cKO:ob/ob mice by postneonatal synoviolin knockout. Black arrows indicate the subcutaneous fat.
Fig. 3C shows a graph indicating body weight changes in syvnl cKO:db/db mice by postneonatal synoviolin knockout.
Fig. 3D shows photographs indicating a state of subcutaneous fat in syvnl cKO:db/db mice by postneonatal synoviolin knockout. Black arrows indicate the subcutaneous fat.
Fig. 4 shows photographs indicating influences of synoviolin gene inhibition with respect to adipocyte differentiation induction of 3T3-L1 cell line. White circles: differentiated fat cells, white rectangle: lipid droplets not normal adipocytes.
Fig. 5A shows a result of PCR indicating that fat-specific synoviolin knockout mice have been generated. PCR products were obtained by amplifying genomic DNA isolated from WAT and BAT of aP2-Cre;Syvnl^{flox/flox} (fat-specific knockout) mice and Syvnl^{flox/flox} mice (control). The PCR products were separated by gel electrophoresis in a conventional manner.
Fig. 5B shows a graph indicating a survival rate of fat-specific synoviolin knockout mice. Survival analysis was performed for aP2-Cre;Syvnl^{flox/flox} mice and control mice (Syvnl^{flox/flox} and Syvnl^{flox/+} mice). Kaplan-Meier curves represent the survival rate for the day of age.
Fig. 5C shows a graph indicating body weight changes in fat-specific synoviolin knockout mice. The body weight was measured from the third day after birth. Adipose KO: aP2-Cre;Syvnl^{flox/flox} (fat-specific knockout) mice. Contorol: Syvnl^{fxox/flox} and Syvnl^{flox/+} mice. Adipose heterozygous: aP2-Cre; Syvnl^{flox/+} mice. The results are shown by the average ± SD. * P<0.01: Comparison of aP2-Cre; Syvnl^{flox/flox} mice with the other mice was performed by post hoc test of ANOVA (Tukey-Kramer method).
Fig. 5D shows a photograph of control mouse and aP2-Cre;Syvnl^{flox/flox} (fat-specific knockout) mouse.
Fig. 5E shows photographs of subcutaneous fats in abdomen of the mice shown in Fig. 5D. White arrows represent the subcutaneous fats.
Fig. 5F shows a photograph of epididymal of the mice shown in Fig. 5D.
Fig. 5G shows photographs of subcutaneous fats in the backs of the mice shown in Fig. 5D. WAT decreased in the aP2-Cre;Syvnl^{flox/flox} mouse (BAT did not decrease).
Fig. 5H shows photomicrographs of adipose tissues in control mouse (left) and aP2-Cre;Syvnl^{flox/flox} mouse (right) of 18th days after birth. Magnification: 400 folds. Scale bar: 50 µm. Black arrow: lipid droplets.
Fig. 6A shows a graph is a graph indicating an influence of LS-102 treatment on body weight change rate. Daily treatment of intraperitoneal injection of DMSO or 50 mg/kg LS-102 to C57BL/6J mice was performed and body weight was measured. The results are represented by the avarage ± SD. * P <0.05: Comparison of C57BL/6J DMSO treated mice and the Student's t-test was performed for C57BL/6J LS-102 treated mice.
Fig. 6B shows a graph indicating an influence of LS-102 treatment on food intake. Average of daily food intake was measured. The results are presented by the avarage ± SD, and the Student's t-test was performed.
Fig. 6C shows a photograph and a graph indicating that fat mass of epididymis has been reduced by LS-102 treatment. DMSO (left) or 50 mg/kg of LS-102 (right) was treated for 57 days to adipose tissues of epididymises in C57BL/6J mice. Results are represented by the avarage ± SD, and Student's t-test (n=4) was performed.
Fig. 6D shows photographs indicating that lipid droplets were reduced by LS-102 treatment. Adipose tissues of epididymises in C57BL/6J mice treated with the DMSO (left) and the 50 mg/kg of LS-102 (right) were stained with HE. Magnification: 400 folds. Scale bar: 50µm. Black arrow: lipid droplets.
Fig. 7 shows a Western blot (left) obtained by observation of synoviolin protein expression level in white adipose tissues of subcutaneous fat of syvnl WT (WT), syvnl cKO (KO), ob/+ mice, and ob/ob mice, and a graph (right) indicating the degree of the expression level based on image analysis.

### DESCRIPTION OF EMBODIMENTS

A screening method for a substance having an anti-obesity action is described herein. The method includes a step for contacting a test substance and a synoviolin-gene-expressing cell; and a step for verifying the effect of the test substance on the synoviolin gene expression, or the effect thereof on synoviolin protein activity. The screening method for a substance having an anti-obesity action is a method for searching a candidate for the substance having an anti-obesity action.

One of anti-obesity activities is a weight-regulating action. The weight-regulating action refers to an action that causes a change in body weight by a significant difference before and after treatment. Weight-regulating action acts through, for example, regulation of the amount of adipose tissue or regulation of the induction of adipocyte differentiation.

The accession number of human synoviolin gene in the public gene database Genbank is AB024690 (SEQ ID NO: 1) .

The nucleotide sequence of the gene encoding human synoviolin is shown in SEQ ID NO: 1. Moreover, proteins other than the protein encoded by this nucleotide sequence, that are highly homologous to the sequence (normally, 70% or more; preferably 80% or more; more preferably 90% or more; and most preferably 95% or more) and have functions of the synoviolin protein, are included in the synoviolin of the present invention.

The term "synoviolin gene" as used herein includes, for example, endogenous genes of other organisms that correspond to a DNA comprising the nucleotide sequence of SEQ ID NO: 1 (such as that of homologs of the human synoviolin gene).

Moreover, the endogenous DNA of other organisms that corresponds to DNA comprising the nucleotide sequence of SEQ ID NO: 1 is generally highly homologous to the DNA of SEQ ID NO: 1. The term "highly homologous" means a homology of 50% or more, preferably 70% or more, more preferably 80% or more, and yet more preferably 90% or more (for example, 95% or more, and further, 96%, 97%, 98%, or 99% or more). This homology can be determined using the mBLAST algorithm (Altschul et al., 1990, Proc. Natl. Acad. Sci. USA 87: 2264-8; Karlin and Altschul, 1993, Proc. Natl. Acad. Sci. USA 90: 5873-7). Moreover, if the DNA is isolated from an organism, it is considered to hybridize with the DNA of SEQ ID NO: 1 under stringent conditions. Here, examples of the "stringent conditions" include "2×SSC, 0.1% SDS, 50°C", "2×SSC, 0.1% SDS, 42°C", and "1×SSC, 0.1% SDS, 37°C". Examples of more stringent conditions include "2×SSC, 0.1% SDS, 65°C", "0.5×SSC, 0.1% SDS, 42°C", and "0.2×SSC, 0.1% SDS, 65°C". Those skilled in the art are capable of appropriately obtaining endogenous genes of other organisms that correspond to the synoviolin gene, based on the nucleotide sequence of the synoviolin gene. In the present specification, proteins (genes) corresponding to synoviolin proteins (genes) in organisms other than humans, or proteins (genes) functionally equivalent to the synoviolin described above, may be simply referred to as "synoviolin protein (gene)".

The "synoviolin protein" of the present invention can be prepared as a natural protein or as a recombinant protein using gene recombination techniques. Natural proteins can be prepared, for example, by a method using affinity chromatography, which employs antibodies against synoviolin protein, from cell (tissue) extracts considered to express the synoviolin protein. In addition, recombinant proteins can be prepared by culturing cells transfected by a DNA encoding the synoviolin protein. The "synoviolin protein" of the present invention is suitably used in, for example, the screening methods described below.

In the present invention, the term "expression" includes "transcription" from genes, "translation" into polypeptides, and the "inhibition of degradation" of proteins. The "expression of synoviolin protein" means the occurrence of the transcription and translation of a gene encoding the synoviolin protein, or the production of the synoviolin protein by such transcription and translation.

The various functions mentioned above can be appropriately evaluated (measured), using general techniques, by those skilled in the art. Specifically, the methods described in the Examples below, or such methods suitably modified, can be performed.

Accordingly, the term "inhibiting the expression and/or function of synoviolin protein" refers to lowering or eliminating the quantity, function, or activity of a synoviolin gene or protein as compared with the quantity, function, or activity of the wild-type synoviolin gene or protein. The term "inhibition" includes the inhibition of either or both of function and expression.

Specifically, ubiquitination means a process for the formation of a polyubiquitin chain via repeated cascade of reactions with enzymes such as ubiquitin-activating enzyme (E1), ubiquitin-conjugating enzyme (E2), and ubiquitin ligase (E3), by which ubiquitin molecules are conjugated in a branched form to a substrate protein. The polyubiquitin chain is formed via an ε-amino group at Lys48 in ubiquitin molecules, and then becomes a degradation signal for the 26S proteasome, leading to the degradation of target proteins.

To confirm influences on synoviolin gene expression or synoviolin protein activity by test substances, such a method as disclosed in WO 2006-137514 can be used.

### Influences of test substances on synoviolin gene expression

A test compound is contacted with cells expressing the synoviolin gene. The "cells" to be used include those derived from a human, mouse, rat, or the like. Microbial cells such as E. coli and yeast, transformed to express synoviolin, can also be used. The "cells expressing the synoviolin gene" may be either cells expressing an endogenous synoviolin gene, or cells expressing a foreign synoviolin gene introduced into the cells. Typically, the cells expressing a foreign synoviolin gene can be produced by introducing an expression vector having the synoviolin gene inserted therein into a host cell. The expression vector can be produced with common genetic engineering techniques.

The test compound to be used in the present method is not specifically limited, but includes, for example, single compounds such as natural compounds, organic compounds, inorganic compounds, proteins, and peptides, as well as compound libraries, expression products from gene libraries, cell extracts, cell culture supernatants, microbial fermentation products, marine organism extracts, plant extracts, and the like.

The "contact" of the test compound with the cell expressing the synoviolin gene is normally performed by adding the test compound to a culture fluid containing cells expressing the synoviolin gene, although it is not limited to this method. If the test compound is a protein or the like, the "contact" can be performed by introducing a DNA vector expressing the protein into the cell.

In the present method, the synoviolin gene expression level is then measured. Herein, the term "gene expression" includes both gene transcription and translation. The gene expression level can be measured with methods known to those skilled in the art.

For example, the gene transcription level can be measured by extracting mRNA from cells expressing the synoviolin gene according to a usual method, and performing Northern hybridization, RT-PCR, a DNA assay method, and the like, using the mRNA as a template. Moreover, the gene translation level can be measured by collecting a protein fraction from cells expressing the synoviolin gene, and by then detecting synoviolin protein expression using electrophoretic methods such as SDS-PAGE. Furthermore, the gene translation level can also be measured by performing Western blotting using an antibody against the synoviolin protein to detect protein expression. The antibody used for detecting the synoviolin protein is not specifically limited as long as the antibody can detect the protein, and, for example, both monoclonal antibodies and polyclonal antibodies can be used.

In the present method, compounds that lower synoviolin expression level as compared to the situation where the test compound is not contacted (control) are selected. The compounds lowering the expression level of synoviolin serve as drugs for anti-obesity.

Furthermore, compounds lowering (suppressing) synoviolin protein activity as compared to the situation where the test compound is not contacted (control) are selected. The compounds lowering (suppressing) the synoviolin protein activity serve as drugs for treating cancer. Influences on synoviolin protein activity include influence on self-ubiquitination of synoviolin protein.

### Influence on self-ubiquitination of synoviolin protein

For example, Japanese Patent Application Publication No. 2008-74753 (Japanese Patent No. 5008932) discloses that plumbagin (2-methyl-5-hydroxy-1, 4-naphthoquinone) and quercetin (2-(3, 4-dihydroxyphenyl)-3,5,7-trihydroxy-4H-1-benzopyrano-4-on) inhibit self-ubiquitination of synoviolin protein. The self-ubiquitination of synoviolin means ubiquitination of protein caused by synoviolin-synoviolin interactions as disclosed in Japanese Patent Application Publication No. 2008-74753. The self-ubiquitination of protein occurs when synoviolin binds to protein.

Influence of synoviolin protein on self-ubiquitination may be confirmed by using a method disclosed in Japanese Patent Application Publication No. 2008-74753 (Japanese Patent No. 5008932). For example, test substances may be added to in vitro self-ubiquitination reaction solution of MBP-Syno ΔTM-His to react at 37°C for 30 minutes. After the reaction, Western blot using anti-HA antibody may be performed to detect ubiquitinated protein. MBP-Syno ΔTM-His means synoviolin in which transmembrane domain has been deleted and which has maltose-binding protein fused to N terminus side thereof and His tag fused to C terminus side thereof.

In the screening method the anti-obesity action is an action of reducing an amount of adipose tissues, or an action of inhibiting a differentiation induction of adipocytes. Namely, according to this, a compound having the action of reducing the amount of adipose tissues or the action of inhibiting the differentiation induction of adipocytes can be screened.

The present invention relates to an anti-obesity drug, namely the therapeutic agent as defined in claim 1. The anti-obesity drug contains a decoy nucleic acid of synoviolinas an active ingredient thereof. The anti-obesity drug has, for example, an action of reducing an amount of adipose tissues, or an action of inhibiting a differentiation induction of adipocytes.

A therapeutic agent which contains an siRNA of synoviolin as an active ingredient is described herein. Nucleic acids having an inhibitory action by means of the RNAi effect are generally referred to as siRNA or shRNA. RNAi is a phenomenon in which, when a short double-stranded RNA (herein abbreviated as "dsRNA") that is composed of a sense RNA comprising a sequence homologous to mRNA of the target gene, and an antisense RNA comprising the complementary sequence thereto, is introduced into a cell, the dsRNA specifically and selectively binds to target gene mRNA and induces its disruption, and efficiently inhibits (suppresses) target gene expression by cleaving the target gene. For example, when a dsRNA is introduced into a cell, the expression of a gene having a sequence homologous to the RNA is suppressed (knocked down). As RNAi is capable of suppressing target gene expression as described above, the technique is attracting attention as a simple gene knockout method replacing conventional gene disruption methods based on homologous recombination, which are complicated and inefficient, and as a method applicable to gene therapy. RNA used for RNAi is not necessarily completely identical to the synoviolin gene or to a partial region of the gene, although it is preferably completely homologous.

siRNA can be designed as follows:
(a) There is no specific limitation to a target region, and any region in the gene encoding synoviolin can be used as a target candidate. For example, in the case of humans, any region in GenBank accession No. AB024690 (SEQ ID NO: 1) can be used as a candidate.
(b) From selected regions, sequences starting with AA, which are 19 to 25 bases long, and preferably 19 to 21 bases long, are selected. For example, sequences having a CG content of 40 to 60% may be selected.
   (a) contacting a test compound with a cell expressing the synoviolin gene;
   (b) measuring synoviolin gene expression in the cell; and
   (c) selecting a compound lowering the expression level as compared to that measured in the absence of the test compound.

An example of synoviolin siRNA is a RNA having one base sequence selected from the following SEQ ID Nos. 2 to 6, complemental base sequences thereof, or a base sequence obtained by replacing, inserting, deleting, or adding one base or two bases with respect to one of these base sequences. The RNA having base sequences expressed by the SEQ ID Nos. 2 to 4 are known as synoviolin siRNA as disclosed in Izumi T, et al., Arthritis Rheum. 2009; 60(1); 63-72., EMBO, Yamasaki S, et al., EMBO J. 2007; 26(1): 113-22. by using experiments. The RNA having base sequences expressed by the SEQ ID Nos. 5 and 6 are known as synoviolin siRNA as disclosed in WO 2005/074988 by using experiments.

SEQ ID NO. 2: 5' -GCUGUGACAGAUGCCAUCA-3'
SEQ ID NO. 3: 5' -GGUGUUCUUUGGGCAACUG-3'
SEQ ID NO. 4: 5' -GGUUCUGCUGUACAUGGCC-3'
SEQ ID NO. 5: 5' -CGUUCCUGGUACGCCGUCA-3'
SEQ ID NO. 6: 5' -GUUUTGGUGACUGGUGCUA-3'

The "RNA having a base sequence obtained by replacing, inserting, deleting, or adding one base or two bases with respect to one of these base sequences" is an RNA having a base sequence obtained by replacing, inserting, deleting, or adding one base or two bases with respect to the one base sequence selected from the SEQ ID Nos. 2 to 6 or the complemental base sequences thereof. One of replacement, insertion, deletion, and addition may be occurred, and two or more may be occurred.

For example, WO 2005-018675 and WO 2005-074988 disclose siRNA to a gene encoding synoviolin, a screening method thereof, and an evaluation method thereof. siRNA to a gene encoding synoviolin can be evaluated by appropriately using the methods disclosed in these publications.

The therapeutic agent of the present invention contains, as an active ingredient, a synoviolin decoy nucleic acid having a base sequence represented by SEQ ID No. 7 or a base sequence obtained by replacing, inserting, deleting, or adding one base or two bases with respect to the base sequence represented by SEQ ID No. 7. The nucleic acid having the base sequence represented by the SEQ ID No. 7 is known as synoviolin decoy nucleic acid as disclosed in Tsuchimochi K, et al., Mol. Cell Biol.2005; 25(16): 7344-56 by examples (SEQ ID NO. 7: 5' -AUGG UGAC UGGU GCUA AGA-3').

The synoviolin decoy nucleic acid and a confirmation method thereof are known as disclosed in, for example, WO 2005-093067 and WO 2005-074988.

A therapeutic agent of which contains, as an active ingredient, a synoviolin antisense nucleic acid is also decribed herein. The synoviolin antisense nucleic acid and a screening method thereof are disclosed in, for example, Japanese Patent Application Publication No. 2009-155204, Re-publication of PCT International Publication No. 2006-137514, and Re-publication of PCT International Publication No. 2005-074988. The synoviolin antisense nucleic acid means a nucleic acid having complemental sequence of synoviolin gene and inhibiting synoviolin gene expression by hybridization to the gene. The antisense nucleic acid can be used to prepare complemental nucleic acid compounds to partial base sequence of a gene encoding synoviolin by synthetic chemical technique. In order to measure whether the nucleic acid compounds efficiently inhibit synoviolin production, screening test may be performed with expression level of the gene as an index. An exemplary antisense nucleic acid compound can suppress, for example, synoviolin expression at least to 50% or less as compared with control.

To inhibit the expression of a specific endogenous gene, methods utilizing antisense technology are well known to those skilled in the art. There are multiple factors by which an antisense nucleic acid inhibits target gene expression. These include inhibition of transcription initiation by triple strand formation, transcription inhibition by hybrid formation at a local open loop structure formed by RNA polymerase, transcription inhibition by hybrid formation with RNA being synthesized, splicing inhibition by hybrid formation at the junction between an intron and an exon, splicing inhibition by hybrid formation at a spliceosome formation site, inhibition of mRNA translocation from the nucleus to the cytoplasm by hybrid formation with mRNA, splicing inhibition by hybrid formation at a capping site or poly-A addition site, inhibition of translation initiation by hybrid formation at a translation initiation factor-binding site, translation inhibition by hybrid formation at a ribosome binding site near the initiation codon, inhibition of peptide chain elongation by hybrid formation in the translated region or polysome binding site of mRNA, inhibition of gene expression by hybrid formation at a nucleic acid-protein interaction site, etc. As described above, antisense nucleic acids inhibit target gene expression by interfering with various processes such as transcription, splicing, or translation (Hirashima and Inoue, "Shin Seikagaku Jikken Koza" [New Biochemistry Experimentation Lectures] 2; Kakusan (Nucleic Acids) IV; Idenshi No Fukusei To Hatsugen [Replication and Expression of Genes]" Edited by The Japanese Biochemical Society, Tokyo Kagaku Dozin, 319-347, 1993).

The antisense nucleic acids described herein may inhibit synoviolin gene expression and/or function by any of the above mechanisms. An antisense sequence designed to be complementary to the untranslated region near the 5'-terminal of mRNA of the synoviolin gene may be considered to be effective in inhibiting the translation of the gene. Moreover, sequences complementary to the coding region or to the untranslated region on the 3' side can also be used. Thus, nucleic acids comprising not only antisense sequences of translated regions of the synoviolin gene, but also those of untranslated regions, are included in the antisense nucleic acids described herein. The antisense nucleic acid to be used is linked to the downstream region of an appropriate promoter, and preferably, a sequence containing a transcription termination signal is connected to the 3' side. A desired animal (cell) can be transformed with the nucleic acid thus prepared using known methods. The sequence of the antisense nucleic acid is preferably complementary to the endogenous-synoviolin gene of the animal (cell) to be transformed or a portion thereof, but the sequence may not be completely complementary as long as the sequence can effectively inhibit gene expression. The transcribed RNA preferably has a complementarity of 90% or more, and most preferably 95% or more, to the transcript of the target gene. In order to effectively inhibit the expression of the target gene (synoviolin) by using an antisense nucleic acid, the antisense nucleic acid is preferably at least 15 bases long but less than 25 bases long. However, antisense nucleic acids described herein are not necessarily limited to this length, and may be 100 bases long or longer, or 500 bases long or longer.

Moreover, ribozymes or DNAs encoding ribozymes can also be used to inhibit synoviolin gene expression. "Ribozyme" refers to an RNA molecule that has a catalytic activity. Ribozymes have various activities. Studies focusing on ribozymes as RNA cleaving enzymes have made it possible to design ribozymes that site-specifically cleave RNA. Some ribozymes such as group I intron ribozymes or the Ml RNA contained in RNase P consist of 400 nucleotides or more, whereas others, called the hammerhead or hairpin ribozymes, have activity domains of about 40 nucleotides (M. Koizumi and E. Ohtsuka, Tanpakushitsu Kakusan Kohso [Protein, Nucleic Acid, and Enzyme], 35: 2191, 1990).

For example, the self-cleavage domain of hammerhead ribozymes cleaves the 3' side of C15 in the G13U14C15 sequence. The base pair formation between U14 and A9 is considered important for the above cleavage activity, and it has been shown that the cleavage may also occur when C15 is replaced with A15 or U15 (M. Koizumi et al., FEBS Lett. 228: 228, 1988). When ribozymes are designed to have substrate-binding sites that are complementary to RNA sequences near target sites, they can be restriction enzyme-like RNA-cleaving ribozymes that recognize the sequence of UC, UU, or UA in target RNA (Koizumi, M. et al., FEBS Lett. 239: 285, 1988; M. Koizumi and E. Ohtsuka, Tanpakushitsu Kakusan Kohso [Protein, Nucleic acid, and Enzyme], 35: 2191, 1990; Koizumi, M. et al., Nucl. Acids Res. 17: 7059, 1989).

Hairpin type ribozymes are also useful for the purpose of the present invention. Such a ribozyme can be found, for example, in the minus strand of the satellite RNA of tobacco ringspot virus (Buzayan, J. M., Nature, 323: 349, 1986). It has been shown that target-specific RNA-cleaving ribozymes can also be produced from hairpin type ribozymes (Kikuchi, Y. and Sasaki, N., Nucleic Acids Res. 19: 6751, 1991; Yo Kikuchi, Kagaku To Seibutsu [Chemistry and Biology] 30: 112, 1992). Thus, the expression of the synoviolin gene of the present invention can be inhibited by specifically cleaving the transcript of the gene.

The expression of endogenous genes can also be suppressed by RNA interference (hereinafter abbreviated as "RNAi") using a double-stranded RNA having the same or similar sequence to the target gene sequence.

The therapeutic agent of the present invention can be administrated orally or parenterally. Transpulmonary administration agent type (e.g. using nebulizer etc.), transnasal administration agent type, transdermal administration agent type (e.g. ointment, creams), injection type, and the like are included in parenteral administration type therapeutic agent of the present invention. The injection type therapeutic agent can be administered to the whole body or locally by intravenous injection such as dripping, intramuscular injection, intraperitoneal injection, hypodermic injection, or the like.

Administration method is appropriately selected depending on age and symptom of a patient. Effective administration amount is 0.1 µg to 100 mg, preferably 1 µg to 10 µg per 1 kg body weight for one administration. However, administration amount of the above therapeutic agent is not restricted to these administration amounts. In a case that nucleic acid such as siRNA is mixed, exemplary amount of the nucleic acid is 0.01 to 10 µg/ml, preferably 0.1 to 1 µg/ml.

The therapeutic agent of the present invention can be formulated in the usual manner and may contain pharmaceutically acceptable carriers or additives. Such carriers or additives include water, pharmaceutically acceptable organic solvents, collagen, polyvinyl alcohol, polyvinylpyrrolidone, carboxyvinyl polymer, carboxymethylcellulose sodium, sodium polyacrylate, sodium alginate, water-soluble dextran, carboxymethyl starch sodium, pectin, methylcellulose, ethylcellulose, xanthan gum, gum arabic, casein, agar, polyethylene glycol, diglycerol, glycerol, propylene glycol, vaseline, paraffin, stearyl alcohol, stearic acid, human serum albumin, mannitol, sorbitol, lactose, surfactant acceptable as pharmaceutical additive, and the like.

The above additive may be selected alone or appropriately in combination from the above additives depending on the pharmaceutical form of the therapeutic agent of the present invention. For example, in a case of being used as an injectable preparation, refined ER stress inducer may be dissolved into a solvent (e.g. saline, buffer solution, glucose solution, or the like), and Tween80, Tween20, gelatin, human serum albumin, or the like may be added to the solution. Alternatively, the therapeutic agent of the present invention may be lyophilized to have a pharmaceutical form of dissolving before use. For example, sugar alcohol or saccharide such as mannitol or glucose can be used as excipient for lyophilization.

The present invention found out that inhibiting expression or activity of synoviolin by generation of synoviolin gene knockout mice is effective for treatment of obesity. Synoviolin gene knockout mice refer to mice in which the normal expression of synoviolin has been inhibited as a result of artificially modifying the sequence of the synoviolin gene region, and as a result thereof, preventing synoviolin from functioning normally in the body.

In addition, a portion or all of synoviolin gene can be modified or deleted. Here, "all" of synoviolin gene refers to a region extending from the 5' end of exon 1 to the 3' end of the final exon of synoviolin genomic DNA. In addition, a "portion" of synoviolin gene refers to a portion of this region of a length required for inhibition of normal expression of synoviolin gene. Moreover, "modified" refers to changing the base sequence of a target region of genomic DNA to another base sequence by substituting, deleting, inserting and/or translocating one or a plurality of nucleotides.

A knockout animal in which a portion or all of synoviolin gene has been modified or deleted can be produced according to a known method. For example, a knockout animal can be produced using a gene targeting method as described in the following examples. In this method, by substituting a region at least containing the start codon of exon 1 of synoviolin gene with another base sequence by homologous recombination, normal expression of synoviolin can be inhibited. In addition, knockout animals used in the screening method of the present invention include not only knockout animals produced according to this method, but also progeny thereof.

The animals targeted for use as knockout animals of the present invention are non-human animals, and there are no particular limitations thereon. Examples include mammals such as cows, pigs, sheep, goats, rabbits, dogs, cats, guinea pigs, hamsters, mice or rats. Rabbits, dogs, eats, guinea pigs, hamsters, mice or rats are preferable for use as experimental animals. In particular, rodents are more preferable, while mice and rats are particularly preferable when considering that a large number of inbred strains have been produced as well as in consideration of techniques such as culturing of fertilized eggs or in vitro fertilization.

A portion of synoviolin gene of a target animal is isolated in order to construct a targeting vector. For example, in the case of producing a knockout mouse, a mouse genomic DNA library is screened for synoviolin gene. A targeting vector for homologous recombination is then constructed using the resulting genomic DNA clone. The genomic DNA clone is not required to be the full length of the gene. All required is cloning of a region required to suppress expression of synoviolin by disrupting synoviolin gene. In addition, the targeting vector can also be produced by a known method, and for example, can be produced by using a commercially available plasmid as a backbone and suitably linking respective fragments consisting of the aforementioned genomic DNA clone, a positive selection marker, a negative selection marker and the like.

The targeting vector produced according to the aforementioned method is then introduced into cells having the ability to form an individual (totipotency), such as fertilized eggs, early embryos or embryonic stem cells (ES cells), by electroporation and the like, followed by selecting those cells in which the target homologous recombination has occurred. Screening is carried out by selecting cells using chemical agents according to a positive-negative selection method. Following selection, those cells in which the target homologous recombination has occurred are confirmed by southern blotting or PCR and the like. Finally, cells for which the desired homologous recombination has been confirmed are introduced into an 8-cell stage embryo or blastocyst harvested from the fallopian tube or uterus during pregnancy.

The aforementioned 8-cell stage embryo or blastocyst is transplanted into an allomother in accordance with ordinary methods. By crossing the germ-line chimeric animals (preferably males) born from the allomother with wild-type animals (preferable females) homologous for the wild-type syvnlgene, a first generation (Fl) can be obtained in the form of heterozygotes in which one of the synvlgenes on a homologous chromosome has been disrupted by homologous recombination. Moreover, by crossing these heterozygotes, a second generation (F2) can be obtained in the form of homozygotes in which both syvnl genes on homologous chromosomes have been disrupted, namely the synvl knockout animals of the present invention. Homozygotes are identified by severing a portion of the body (such as the tail), extracting DNA and determining genotype by southern blotting or PCR and the like.

Although the following provides a more detailed explanation of the present invention using the following examples, the present invention is not limited by these examples.

### EXAMPLES

### <Generation example 1: Generation of synoviolin knockout mice>

The design drawing of a gene targeting vector used to produce synoviolin knockout mice of the present example is shown in FIG. 1A. In the drawing, the structures of a normal synoviolin gene (Wild allele), targeting vector for producing a synoviolin knockout mouse (Targeting vector), allele that has undergone homologous recombination (Targeted allele), allele containing a loxP sequence (Flox allele) and allele from which loxP-Exons 2 to 14-loxP has been deleted (Deleted allele) are respectively schematically shown starting at the top in that order.

A region upstream from exon 1 and downstream from exon 16 of mouse synoviolin gene in the form of a 14.8 kb gene region was used to construct the targeting vector. A Neo resistance gene interposed between FRT sequences was inserted between exon 1 and exon 2. In addition, loxP sequences were introduced upstream from exon 2 and downstream from exon 14.

The aforementioned targeting vector was introduced into ES cells. Clones having an allele in which the target homologous recombination has occurred were selected by confirming removal of the loxP-exon-loxP sequence by Cre treatment and removal of the FRT-neomycin-FRT sequence by FLP treatment using the lengths of the PCR products.

Chimeric mice were obtained by introducing the aforementioned ES cell clones that had undergone homologous recombination into mouse embryos in accordance with a known method (e.g., EMBO J 16:1850-1857). Moreover, these chimeric mice were crossed with wild-type C57BL/6 mice to acquire mice in which the neomycin sequence had been removed. In addition, since loxP sequences between exons and the long arm incorporated in the targeting vector have the possibility of being lost during homologous recombination, their presence was confirmed by PCR.

The resulting neomycin-removed mice were crossed with CAG-CreER mice to obtain CAG-Cre-ER; *syvnl*^{flox/flox} mice.

### (Example 1: Phenotype of postneonatal synoviolin knockout)

In order to elucidate the postneonatal function of synoviolin, tamoxifen was administered to synoviolin knockout mice (*CAG*-Cre-ER; *syvnl*^{flox/flox} mice) inducible by tamoxifen (Tam) to induce removal of loxP-Exon-loxP sequence (CAG-CreER (+) syvnl^{flox/flox}). In addition, as a control, each group of C57BL/6J (solvent control, tamoxifen administration) and (CAG-CreER (-) syvnl^{flox/flox)} (solvent control, tamoxifen administration) was provided.

Specifically, 7-8 weeks after birth, C57BL/6J mice, homozygous *syno*^{flox/flox} mice which are deficient in Cre induction gene *(syvnl* WT), and *CAG*-Cre;*syno*^{flox/flox} mice *(syvnl* cKO) were intraperitoneally administered with 125 mg/kg of tamoxifen solution per day for 5 consecutive days. Knockout of synoviolin by administration of tamoxifen was confirmed by PCR of synoviolin on genome, real-time PCR of synoviolin mRNA, western brotting of synoviolin protein, and the like (Figs. 1B to 1D). Syvnl^{flox/flox} mice were controls.

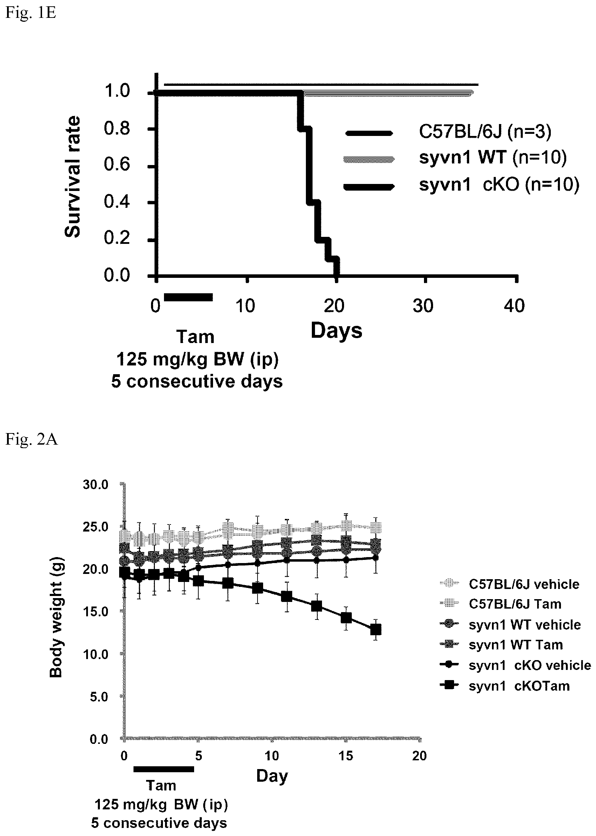

Kaplan-Meier curve was made. The Kaplan-Meier curve indicates survival rates of the C57BL/6J mice (n=3), the *syvnl* WT mice (n=10), and the *syvnl* cKO mice (n=10) after the administration of tamoxifen with respect to age in day. The results are shown in Fig. 1E.

The *syvnl* cKO mice could not survive, and all of them died up to 21 days after Tam administration. On the other hand, C57BL/6J mice and the *syvnl* WT mice were alive (Fig. 1E).

### (Example 2-1: Change in body weight by synoviolin knockout)

7-8 weeks after birth, C57BL/6J mice, homozygous *syno*^{flox/flox} mice *(syvnl* WT), and *CAG*-Cre; *syno*^{flox/flox} mice *(syvnl* cKO) were intraperitoneally administered with 125 mg/kg of tamoxifen solution or control solution per day for 5 consecutive days. The results were shown in Fig. 2A.

Fig. 2A shows that significant decrease in body weight was observed 1 week after the Tam administration in the group of *syvnl* cKO mice and body weights thereof were decreased up to almost half those of the group of *syvnl* WT mice in breeding dates dependent manner. On the other hand, in any of the control groups, reduction in body weight was not observed.

In order to examine whether syvnl cKO mice have an eating disorder and/or malnutrition in absorption stage, the following three examinations were carried out.

### (Example 2-2: Influence on food intake by synoviolin knockout)

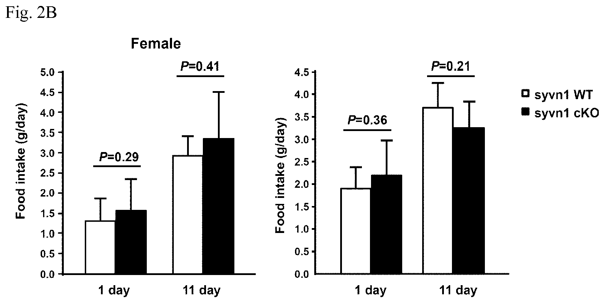

In order to examine whether reduction of body weights in the *syvnl* cKO mice is caused by reduction of food-intake, daily food intake was measured. Fig. 2B shows averages of daily food intake of 1 day after the tamoxifen administration and 11 days after the tamoxifen administration.

Fig. 2B shows that there is no difference between the food intake of the syvnl cKO mice and that of the control mice. This result suggests that the reduction of body weights in the *syvnl* cKO mice is not caused by eating disorder or mere weakness.

### (Example 2-3: Influence on nutrition and functions of liver and kidney by synoviolin knockout)

Further, some biomarkers for nutrition and functions of liver and kidney, which include total protein, albumin, glucose, triglycerides, total cholesterol, AST, ALT, BUN, and Cr, were examined by serum biochemical experiments. The results are shown in Table 1 below. As shown in Table 1, there is no significant change in the two groups.

**[Table 1]**

| | syno WT | | syno cKO | |
|---|---|---|---|---|
| | mean | SD | mean | SD |
| TP(g/dL) | 7.2 | 0.0 | 5.7 | 1.3 |
| ALB(g/dL) | 7.2 | 0.0 | 5.4 | 1.6 |
| BUN(mg/dL) | 32.8 | 4.2 | 29.1 | 1.2 |
| CRE(mg/dL) | 0.1 | 0.0 | 0.1 | 0.0 |
| Na(mEq/L) | 181.3 | 4.6 | 169.7 | 12.7 |
| K(mEq/L) | 7.5 | 0.5 | 8.1 | 0.7 |
| Cl(mEq/L) | 85.3 | 4.6 | 87.7 | 2.5 |
| Ca(mg/dL) | 40.0 | 0.0 | 30.0 | 8.7 |
| IP(mg/dL) | 6.9 | 0.9 | 8.2 | 1.2 |
| AST(IU/L) | 64.0 | 8.0 | 132.3 | 66.9 |
| ALT(IU/L) | 112.0 | 42.3 | 91.7 | 58.4 |
| LDH(IU/L) | 429.3 | 18.5 | 329.7 | 60.2 |
| AMY(IU/L) | 2736.0 | 251.5 | 2600.7 | 115.4 |
| r-GT(IU/L) | 24.0 | 0.0 | 18.0 | 5.2 |
| T-CHO(mg/dL) | 106.7 | 4.6 | 99.0 | 16.8 |
| TG(mg/dL) | 138.7 | 30.3 | 78.7 | 15.8 |
| HDL-C(mg/dL) | 64.0 | 0.0 | 61.3 | 5.5 |
| T-BIL(mg/dL) | 0.1 | 0.0 | 0.1 | 0.0 |
| GLU(mg/dL) | 189.3 | 12.2 | 213.3 | 27.5 |

### (Example 2-4: Influence on adipose tissue by synoviolin knockout)

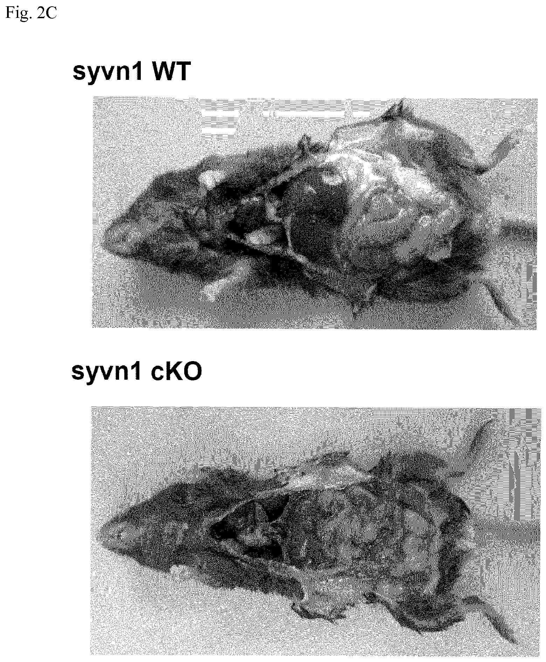

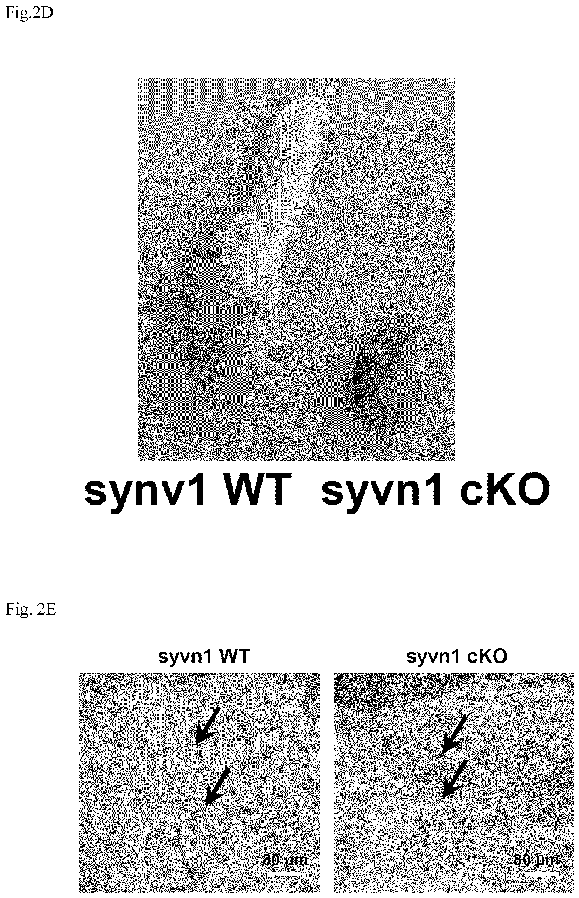

Then, adipose tissues of *syvnl* cKO mice were analyzed with the naked eye and a microscope. Laparotomy was performed for *syvnl* WT mouse (left side) and *syvnl* cKO mouse (right side) 16 days after the tamoxifen administration to observe the adipose tissues thereof. The results were shown in Figs. 2C and 2D.

Fig. 2C shows that substantial amount of food residue has been remained in intestine of the *syvnl* cKO mouse, and any histological abnormality has not been found. In addition, reduction of subcutaneous fat has been found in the *syvnl* cKO mouse.

In addition, Fig. 2D shows that the white adipose tissue (WAT) has been significantly reduced in the *syvnl* cKO mouse. No white adipose tissue was observed particularly in the epididymis thereof.

### (Example 2-5: Influence on lipid droplet by synoviolin knockout)

In order to examine influence on inside of adipose tissue by synoviolin knockout, visceral adipose tissues of *syvnl* WT mouse and *syvnl* cKO mouse were stained with hematoxylin-eosin and observed. Histological analysis was performed by using barely remaining adipose tissue in the *syvnl* cKO mouse. The results were shown in Fig. 2E.

Fig. 2E shows that lipid droplets have been reduced in the *syvnl* cKO mouse. This reduction of the lipid droplets was observed only in the *syvnl* cKO mouse and no reduction of the lipid droplets was observed in the *syvnl* WT mouse.

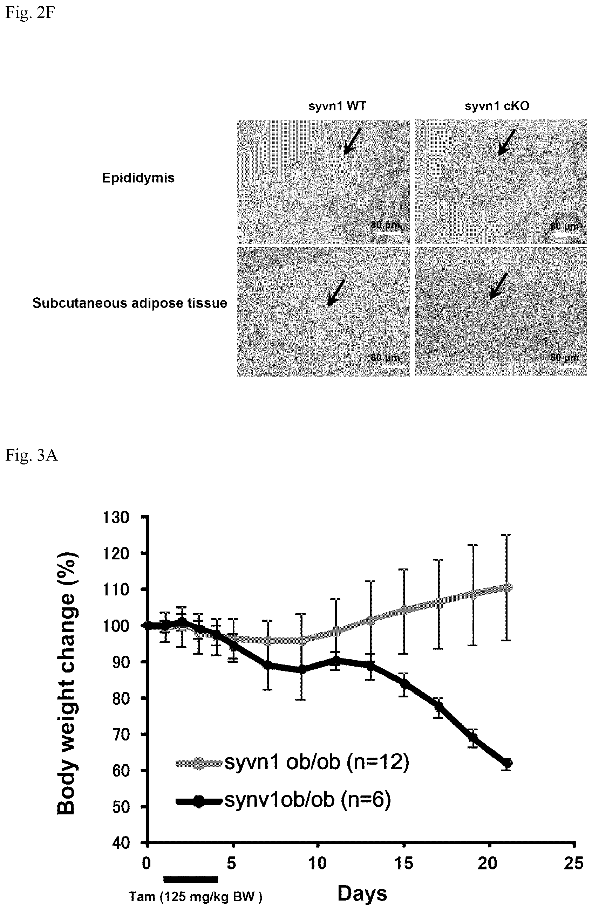

Then, epididymis and subcutaneous of the *syvnl* WT mouse and the *syvnl* cKO mouse were observed in a similar manner. The results were shown in Fig. 2F. Fig. 2F shows photomicrographs showing states of lipid droplets of the epididymis (upper diagrams) and the subcutaneous (lower diagrams) in postneonatal synoviolin conditional knockout mice. Black arrows indicate the lipid droplets. Magnification is 400 folds.

It has been reported that increase in sizes of adipocytes and increase in the number of adipocytes caused by differentiation of preadipocytes are phenomena observed in the beginning of progression of obesity (Faust et al., 1978; Jo et al., 2009), and that inability of storing excessive energy in adipose tissue relates to insulin resistance and metabolic complications (Tan and Vidal-Puig, 2008 Virtue and Vidal-Puig, 2008). Accordingly, anti-obesity action by expression inhibition or function inhibition of synoviolin was strongly suggested.

### (Example 3: Effect of synoviolin knockout in ob/ob mice and db/db mice)

Whether synoviolin knockout induces decrease in body weights of obese mice under a condition of constant intake of high fat diet was verified.

Firstly, by using ob/ob (abnormality of leptin gene) mice and db/db (abnormality of leptin receptor gene) mice (both are overeating due to inability of central feeding and are generally used as models of obesity, diabetes, metabolic syndrome, and the like), which are typical obese mice, these mice were crossed with synoviolin conditional knockout mice to acquire mice having ob or db gene homo, and also synoviolin gene homo, namely, completely knockoutable mice. The following are genotypes of these mice. *CAG*-Cre-ER; *syvnl*^{flox/flox}: *ob*/*ob* mice *(syvnl* cKO:*ob*/*ob* mice), *syvnl*^{flox/flox}: *ob*/*ob* mice *(syvnl* WT:*ob*/*ob* mice), *CAG*-Cre-ER; *syvnl*^{flox/flox}:*db*/*db* mice *(syvnl* cKO:*db*/*db* mice), and *syvnl*^{flox/flox}: *db*/*db* mice *(syvnl* WT: *db*/*db* mice).

As a specific procedure, firstly, *CAG*-Cre-ER;*syvnl*^{flox/flox} mice were crossed with *ob*/+ mice and *db*/+ mice to generate the *CAG*-Cre-ER; *syvnl*^{flox/flox}: *ob*/*ob* mice and the *CAG*-Cre-ER; *syvnl*^{flox/flox}: *db*/*db* mice.

In secondary mating, *CAG*-Cre-ER; *syvnl*^{flox/+}:*ob*/*+* mice were crossed to generate the *CAG*-Cre-ER;*syvnl*^{flox/flox}:*ob*/*ob* mice *(syvnl* cKO:*ob*/*ob*) and the *syvnl*^{flox/flox}:*ob*/*ob* mice *(syvnl* WT:*ob*/*ob)* . Similarly, in secondary mating, *CAG*-Cre-ER; *syvnl*^{flox/+}: *db*/*+* mice were crossed to generate the *CAG*-Cre-ER; *syvnl*^{flox/flox}: *db*/*db* mice *(syvnl* cKO:*db*/*db)* and the *syvnl*^{flox/flox}:*db*/*db* mice (*syvnl* WT: *db*/*db) .*

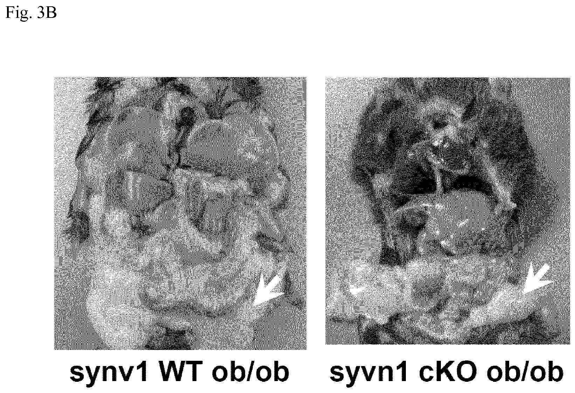

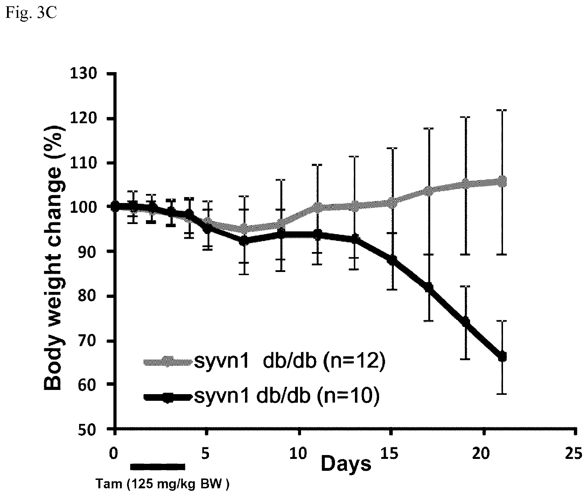

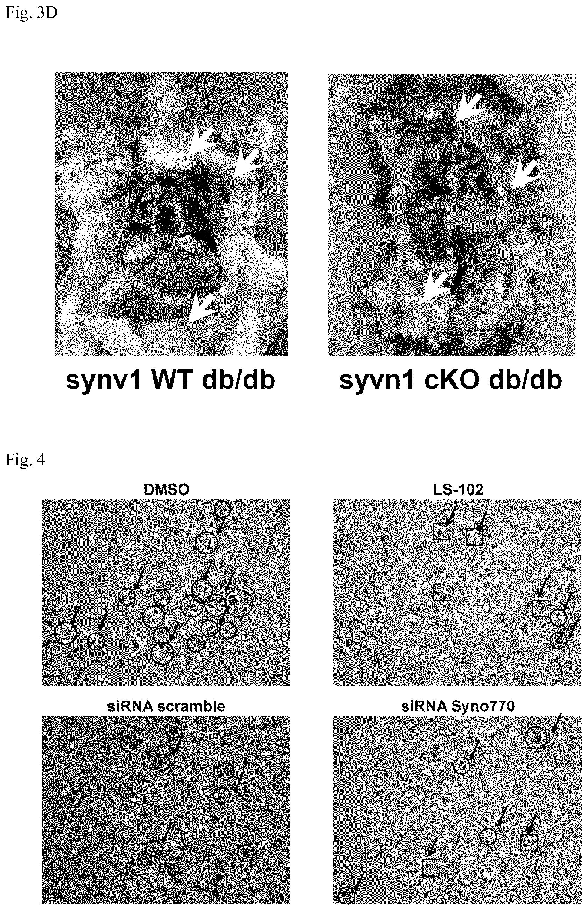

7-8 weeks after birth, these mice (*syvnl* cKO:*ob*/*ob, syvnl* WT:*ob*/*ob, syvnl* cKO:*db*/*db,* and *syvnl* WT:*db*/*db*) were intraperitoneally administered with 125 mg/kg of tamoxifen (Tam) solution per day for 5 consecutive days. Body weights were measured every day after the tamoxifen administration to obtain change of rate in the body weights. The results were shown in Figs. 3A and 3C. In addition, adipose tissues of the *syvnl WT:ob*/*ob* mice and the *syvnl* WT:*db*/*db* mice, and of the *syvnl* cKO*:ob*/*ob* mice and the *syvnl* cKO:*db*/*db* mice 25 days after the tamoxifen administration were observed by dissection. The results were shown in Figs. 3B and 3D.

As shown in Figs. 3A and 3C, the body weights of the *syvnl* WT:*ob*/*ob* mice and the *syvnl* WT:*db*/*db* mice were increased up to 130% after the tamoxifen administration. On the other hand, the body weights of the *syvnl cKO:ob*/*ob* mice and the *syvnl cKO:db*/*db* mice were significantly reduced by approximately half of initial weights of the examination. Namely, these figures show that the postneonatal synoviolin knockout cause the reduction of body weights of the *ob*/*ob* mice and the *db*/*db* mice.

As shown in Figs. 3B and 3D, the volume of fat in the *syvnl* cKO:ob/ob mice and the *syvnl* cKO:*db*/*db* mice was obviously decreased as compared to those of the *syvnl* WT:*ob*/*ob* mice and the *syvnl* WT:*db*/*db* mice. Namely, these figures show that the postneonatal synoviolin knockout cause the reduction of white fat cells of the *ob*/*ob* mice and the *db*/*db* mice.

Considering in combination with the data (Fig. 2B) indicating no difference in food intake, these results indicate that reduction in body weight due to deficiency of synoviolin is a dominant resistance against obesity induced by inactivation of leptin signaling in the level of central nervous system and/or high energy intake. Namely, it can be considered that the deficiency of synoviolin acts on energy consumption in peripheral adipocytes rather than the central nervous system.

### (Example 4: Effect of synoviolin expression inhibition and ubiquitination activity inhibition on obesity)

3T3-L1 cells, which are preadipocytes of mice, were cultivated in Dulbecco's Modified Eagle Medium (DMEM; High Glucose) containing 10% fetal bovine serum (FBS) for 3 days after reaching confluent state. 500µM of isobutylmethylxanthine (IBMX), 1 µM of Dexamethasone, and 5 µg/mL of Insulin were added to induce differentiation. At the same time, 10 µM of LS-102 (ubiquitination activity inhibitor of synoviolin) or DMSO was added. After cultivation for 3 days, the medium was replaced with medium containing 4 µg/mL of Insulin and 10 µM of LS-102 or DMSO was added. After cultivation for 3 days, the medium was replaced with DMEM (High Glucose) containing 10% FBS to cultivate for 3 days. For siRNA, 200 pmol of siRNA Syno770 (sense strand consists of the following SEQ ID NO: 2) was introduced by the Lipofectamine2000 two days before induction of differentiation.
SEQ ID No. 2: 5' -GCUGUGACAGAUGCCAUCA-3'

After the cultivated 3T3-L1 cells were washed with PBS(-) (Phosphate Buffered Saline solution from which magnesium and calcium had been eliminated), the cells were fixed by 10% formalin. The cells were washed with the PBS(-) to be replaced with 60% isopropanol. After staining for 20 minutes with 18 mg/ml of Oil Red O (solvent Isopropanol), the cells were washed with 60% isopropanol and PBS(-) and observed by a microscope. The results were shown in Fig. 4.

Fig. 4 suggests that less adipose cells have been differentiated in the cells in which siRNA has inhibited the synoviolin gene activity as compared to a control, and that the differentiation has been suppressed. Further, lipid droplets not being normal adipocytes in the form of annular ring were observed. The above results indicate that the inhibition of synoviolin gene expression and the inhibition of self-ubiquitination of synoviolin protein are effective for prevention or treatment of obesity.

### (Example 5: Fat reduction in mice in which synoviolin has been knocked out specifically in fat)

In order to confirm whether synoviolin gene (SYVNl) knockout directly targets white adipose tissue, synoviolin knockout mice (*aP2*-Cre; *Syvnl*^{flox/flox} mice: adipose KO) in which synoviolin had been knocked out specifically in fat were generated.

In order to generate the synoviolin knockout mice in which synoviolin had been knocked out specifically in fat, firstly, Syvnl^{flox/flox} mice were crossed with fatty acid-binding protein 4 (aP2)-Cre mice (Jackson Immunoresearch Laboratories) to acquire compound heterozygote including *aP2*-Cre-ER; Syvnl^{flox/+} mice. Secondly, *aP2*-Cre; *Syvnl*^{flox/+} mice were crossed with Syvnl^{flox/flox} mice as the second mating to acquire *aP2*-Cre; *Syvnl*^{flox/flox} mice. Mice having genotype of Syvnl^{flox/flox} or Syvnl^{flox/+} and being deficient in Cre transgene are referred to as control mice.

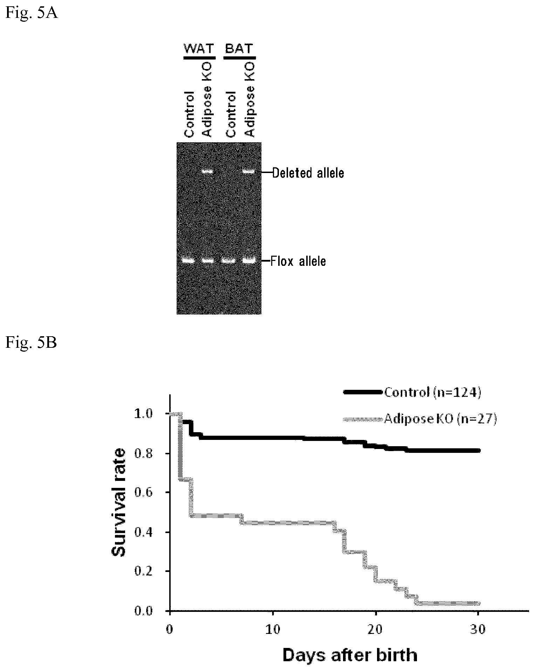

Occurrence of *Syvnl* knockout via Cre recombinase in WAT (white adipose tissue) and BAT (brown adipose tissue) of the acquired *aP2*-Cre; *Syvnl*^{flox/flox} mice was confirmed by PCR (Fig. 5A).

When a survival rate of the *aP2*-Cre; *Syvnl*^{flox/flox} mice was checked, among more than 200 litters, almost all of the *aP2*-Cre;*Syvnl*^{flox/flox} mice died by 24 days after birth (Fig. 5B).

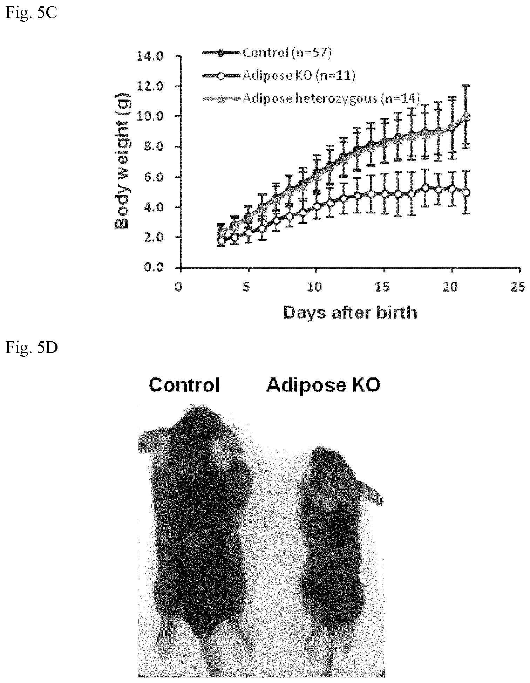

In addition, when change in body weights of the *aP2*-Cre;*Syvnl*^{flox/flox} mice was observed, as compared to the control mice (*Syvnl*^{flox/flox} mice and Syvnl^{flox/+} mice) and *aP2-*Cre; *Syvnl*^{*flox*/+} mice, the body weights of the *aP2-*Cre;*Syvnl*^{flox/flox} mice decreased approximately by half (Figs. 5C and 5D).

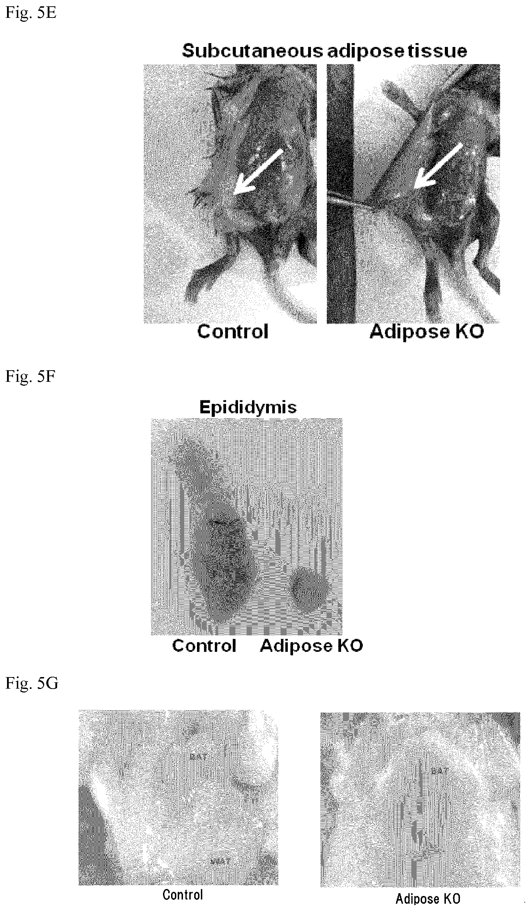

Further, when adipose tissues of the *aP2-*Cre; *Syvnl*^{flox/flox} mice were observed, significant decrease in WAT was observed (Figs. 5E and 5F). On the other hand, no decrease in BAT of the *aP2*-Cre;*Syvnl*^{flox/flox} mice was observed (Fig. 5G).

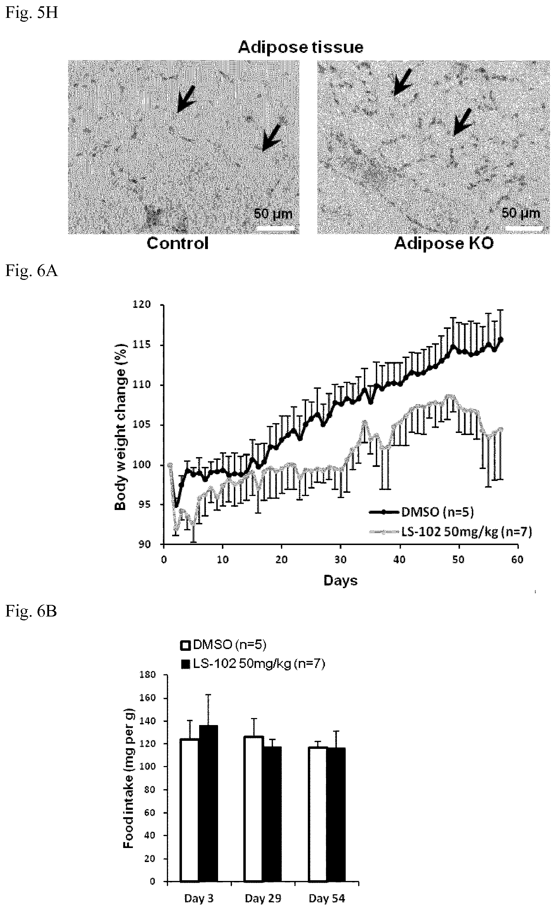

In order to observe tissue sections of the *aP2-*Cre;*Syvnl*^{flox/flox} mice, staining with hematoxylin-eosin (HE) was performed. Large number of lipid droplets were decreased as compared to those of control mice (Fig. 5H).

These results suggest that synoviolin directly targets WAT in body weight control.

### (Example 6: Increase in body weight and decrease in fat amount by selective inhibitor LS-102 of synoviolin)

LS-102 has previously been shown to be a selective inhibitor of E3 ubiquitin ligase activity of synoviolin (SYVNl) (Yagishita, N., et al. Int. J. Mol. Med. 30, 1281- 1286 (2012).) Therefore, whether pharmacological inhibition of SYVNl improves obesity was examined.

Firstly, solvent (DMSO) as a control or 50 mg/kg body weight of LS-102 was administered to C57BL/6J mice, and body weights thereof were measured for approximately 2 months (Fig. 6A). As a result, increase in body weight caused by food intake was inhibited in mice treated with LS-102. No influence of the LS-102 on the food intake existed (Fig. 6B).

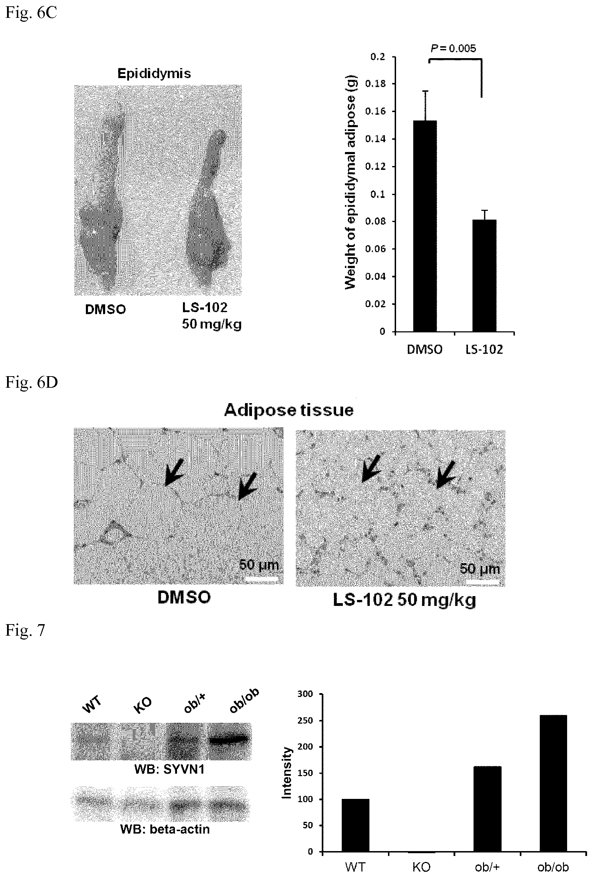

When fat in WAT of epididymis was observed and measured by dissecting the mice treated with the LS-102, reduction of fat mass was observed (Fig. 6C).

Further, when the tissue sections of the mice were observed, lipid droplets were also decreased in the LS-102 treated mice as compared to the control mice (Fig. 6D) .

These results strongly suggest that the LS-102 inhibits obesity via inhibition of SYVNl.

### (Example 7: Applicability of synoviolin as a biomarker)

The example 3 indicated that reduction in body weight and white fat cells are caused by knockout of synoviolin in *ob*/*ob* mice which are obese mice. Therefore, whether synoviolin can be used as a biomarker of obesity was confirmed subsequently. Cell extracts obtained from white adipose tissue (WAT) in subcutaneous of syvnl WT mice, syvnl cKO mice, ob/+ mice, and ob/ob mice were prepared. Western blotting was performed by using anti-synoviolin (SYVNl) antibody and anti-beta-actin antibody (for internal standard). The results are shown in Fig. 7. Fig. 7 shows a western blot indicating synoviolin protein expression in the subcutaneous white adipose tissues of the *ob*/+ mice and the *ob*/*ob* mice (left diagram) and a graph indicating intensity of expression based on image analysis (right diagram).

No synoviolin protein expression was confirmed in the syvnl cKO mice. On the other hand, synoviolin protein expression level was increased in the ob/ob mice as compared to the syvnl WT mice. From the increase in synoviolin expression level in the obese mice, applicability of synoviolin as a biomarker was found.

### INDUSTRIAL APPLICABILITY

According to the screening method described herein, a pharmaceutical composition which is effective for prevention or treatment of obesity can be screened by using inhibition of synoviolin functions as an index.

Further, the anti-obesity drug of the present invention can inhibit differentiation of adipocytes without food-deprivation by inhibiting synoviolin expression or self-ubiquitination of synoviolin protein, can regulate adipose tissue mass and body weight, and is valuable as novel type of anti-obesity drug different from the conventional appetite suppressant or digestive absorption inhibitors.

### SEQUENCE LISTING FREE TEXT

| | |
|---|---|
| Sequence 2: | Synthetic RNA |
| Sequence 3: | Synthetic RNA |
| Sequence 4: | Synthetic RNA |
| Sequence 5: | Synthetic RNA |
| Sequence 6: | Synthetic RNA |
| Sequence 7: | Synthetic RNA |

### SEQUENCE TABLE

12-326P.Seq_ST25.txt

## Claims

1. A therapeutic agent containing a decoy nucleic acid of synoviolin as an active ingredient for use in treatment or prevention of obesity, wherein the decoy nucleic acid of synoviolin has a base sequence represented by sequence ID No. 7, or a base sequence obtained by replacing, inserting, deleting, or adding one base or two bases with respect to the base sequence represented by the sequence ID No. 7.

## Patentansprüche

1. Therapeutisches Mittel, umfassend eine Köder-Nukleinsäure von Synoviolin als aktiven Bestandteil zur Verwendung bei der Behandlung oder zur Vorbeugung von Fettleibigkeit, wobei die Köder-Nukleinsäure von Synoviolin eine Basensequenz, die durch Sequenz ID Nr. 7 dargestellt ist, oder eine Basensequenz, die durch Ersetzen, Einfügen, Deletieren oder Hinzufügen einer Base oder zwei Basen in Bezug auf die Basensequenz, die durch die Sequenz ID Nr. 7 dargestellt ist, erhalten wird, aufweist.

## Revendications

1. Agent thérapeutique contenant un acide nucléique leurre de la synovioline en tant que principe actif pour utilisation dans le traitement ou la prévention de l'obésité, dans lequel l'acide nucléique leurre de la synovioline a une séquence de bases représentée par SEQ ID NO : 7, ou une séquence de bases obtenue par remplacement, insertion, délétion, ou addition d'une base ou de deux bases par rapport à la séquence de bases représentée par la séquence SEQ ID NO : 7.


### SEQUENCE LISTING

<110> Nakajima, Toshihiro
<120> Method for screening a compound having an effect of prevention and treatment of obesity
<130> 12-326P
<160> 7
<170> PatentIn version 3.5
<210> 1
   <211> 3374
   <212> DNA
   <213> Homo sapiens
<220>
   <221> CDS
   <222> (403)..(2256)
<400> 1
   gccctttctt atgagcatgc ctgtgttggg ttgacagtga gggtaataat gacttgttgg     60
   ttgattgtag atatagggct ctcccttgca aggtaattag gctccttaaa ttacctgtaa    120
   gattttcttg ccacagcatc cattctggtt aggctggtga tcttctgagt agtgatagat    180
   tggttggtgg tgaggtttac aggtgttccc ttctcttact cctggtgttg gctacaatca    240
   ggtggcgtct agagcagcat gggacaggtg ggtaagggga gtcttctcat tatgcagaag    300
   tgatcaactt aaatctctgt cagatctacc tttatgtagc ccggcagtcg cgcggattga    360
   gcgggctcgc ggcgctgggt tcctggtctc cgggccaggg ca atg ttc cgc acg       414
   gca gtg atg atg gcg gcc agc ctg gcg ctg acc ggg gct gtg gtg gct      462
   cac gcc tac tac ctc aaa cac cag ttc tac ccc act gtg gtg tac ctg      510
   acc aag tcc agc ccc agc atg gca gtc ctg tac atc cag gcc ttt gtc      558
   ctt gtc ttc ctt ctg ggc aag gtg atg ggc aag gtg ttc ttt ggg caa      606
   ctg agg gca gca gag atg gag cac ctt ctg gaa cgt tcc tgg tac gcc      654
   gtc aca gag act tgt ctg gcc ttc acc gtt ttt cgg gat gac ttc agc      702
   ccc cgc ttt gtt gca ctc ttc act ctt ctt ctc ttc ctc aaa tgt ttc       750
   cac tgg ctg gct gag gac cgt gtg gac ttt atg gaa cgc agc ccc aac       798
   atc tcc tgg ctc ttt cac tgc cgc att gtc tct ctt atg ttc ctc ctg       846
   ggc atc ctg gac ttc ctc ttc gtc agc cac gcc tat cac agc atc ctg       894
   acc cgt ggg gcc tct gtg cag ctg gtg ttt ggc ttt gag tat gcc atc       942
   ctg atg acg atg gtg ctc acc atc ttc atc aag tat gtg ctg cac tcc       990
   gtg gac ctc cag agt gag aac ccc tgg gac aac aag gct gtg tac atg      1038
   ctc tac aca gag ctg ttt aca ggc ttc atc aag gtt ctg ctg tac atg      1086
   gcc ttc atg acc atc atg atc aag gtg cac acc ttc cca ctc ttt gcc      1134
   atc cgg ccc atg tac ctg gcc atg aga cag ttc aag aaa gct gtg aca      1182
   gat gcc atc atg tct cgc cga gcc atc cgc aac atg aac acc ctg tat      1230
   cca gat gcc acc cca gag gag ctc cag gca atg gac aat gtc tgc atc      1278
   atc tgc cga gaa gag atg gtg act ggt gcc aag aga ctg ccc tgc aac      1326
   cac att ttc cat acc agc tgc ctg cgc tcc tgg ttc cag cgg cag cag      1374
   acc tgc ccc acc tgc cgt atg gat gtc ctt cgt gca tcg ctg cca gcg      1422
   cag tca cca cca ccc ccg gag cct gcg gat cag ggg cca ccc cct gcc      1470
   ccc cac ccc cca cca ctc ttg cct cag ccc ccc aac ttc ccc cag ggc      1518
   ctc ctg cct cct ttt cct cca ggc atg ttc cca ctg tgg ccc ccc atg      1566
   ggc ccc ttt cca cct gtc ccg cct ccc ccc agc tca gga gag gct gtg      1614
   gct cct cca tcc acc agt gca gca gcc ctt tct cgg ccc agt gga gca      1662
   gct aca acc aca gct gct ggc acc agt gct act gct gct tct gcc aca      1710
   gca tct ggc cca ggc tct ggc tct gcc cca gag gct ggc cct gcc cct      1758
   ggt ttc ccc ttc cct cct ccc tgg atg ggt atg ccc ctg cct cca ccc      1806
   ttt gcc ttc ccc cca atg cct gtg ccc cct gcg ggc ttt gct ggg ctg      1854
   acc cca gag gag cta cga gct ctg gag ggc cat gag cgg cag cac ctg      1902
   gag gcc cgg ctg cag agc ctg cgt aac atc cac aca ctg ctg gac gcc      1950
   gcc atg ctg cag atc aac cag tac ctc acc gtg ctg gcc tcc ttg ggg      1998
   ccc ccc cgg cct gcc act tca gtc aac tcc act gag ggg act gcc act      2046
   aca gtt gtt gct gct gcc tcc tcc acc agc atc cct agc tca gag gcc      2094
   acg acc cca acc cca gga gcc tcc cca cca gcc cct gaa atg gaa agg      2142
   cct cca gct cct gag tca gtg ggc aca gag gag atg cct gag gat gga      2190
   gag ccc gat gca gca gag ctc cgc cgg cgc cgc ctg cag aag ctg gag     2238
   tct cct gtt gcc cac tga cactgcccca gcccagcccc agcctctgct            2286
   cttttgagca gccctcgctg gaacatgtcc tgccaccaag tgccagctcc ctctctgtct   2346
   gcaccaggga gtagtacccc cagctctgag aaagaggcgg catcccctag gccaagtgga   2406
   aagaggctgg ggttcccatt tgactccagt cccaggcagc catggggatc tcgggtcagt   2466
   tccagccttc ctctccaact cttcagccct gtgttctgct ggggccatga aggcagaagg   2526
   tttagcctct gagaagccct cttcttcccc cacccctttc caggagaagg ggctgcccct    2586
   ccaagcccta cttgtatgtg cggagtcaca ctgcagtgcc gaacagtatt agctcccgtt   2646
   cccaagtgtg gactccagag gggctggagg caagctatga acttgctcgc tggcccaccc    2706
   ctaagactgg tacccatttc cttttcttac cctgatctcc ccagaagcct cttgtggtgg   2766
   tggctgtgcc ccctatgccc tgtggcattt ctgcgtctta ctggcaacca cacaactcag   2826
   ggaaaggaat gcctgggagt gggggtgcag gcgggcagca ctgagggacc ctgccccgcc   2886
   cctcccccca ggcccctttc ccctgcagct tctcaagtga gactgacctg tctcacccag    2946
   cagccactgc ccagccgcac tccaggcaag ggccagtgcg cctgctcctg accactgcaa   3006
   tcccagcgcc caaggaaggc cacttctcaa ctggcagaac ttctgaagtt tagaattgga   3066
   attacttcct tactagtgtc ttttggctta aattttgtct tttgaagttg aatgcttaat   3126
   cccgggaaag aggaacagga gtgccagact cctggtcttt ccagtttaga aaaggctctg   3186
   tgccaaggag ggaccacagg agctgggacc tgcctgcccc tgtcctttcc ccttggtttt   3246
   gtgttacaag agttgttgga gacagtttca gatgattatt taatttgtaa atattgtaca   3306
   aattttaata gcttaaattg tatatacagc caaataaaaa cttgcattaa caaaaaaaaa   3366
   aaaaaaaa                                                            3374
<210> 2
   <211> 19
   <212> RNA
   <213> **Synthetic RDA**
<400> 2
   gcugugacag augccauca 19
<210> 3
   <211> 19
   <212> RNA
   <213> Synthetic RDA
<400> 3
   gguguucuuu gggcaacug 19
<210> 4
   <211> 19
   <212> RNA
   <213> Synthetic RDA
<400> 4
   gguucugcug uacauggcc 19
<210> 5
   <211> 19
   <212> RNA
   <213> Synthetic RDA
<400> 5
   cguuccuggu acgccguca 19
<210> 6
   <211> 19
   <212> DNA
   <213> Synthetic RDA
<400> 6
   guuutgguga cuggugcua 19
<210> 7
   <211> 19
   <212> RNA
   <213> Artificial Sequence
<220>
   <223> Synthetic RNA
<400> 7
   auggugacug gugcuaaga 19